# EUROPÄISCHE PATENTANMELDUNG

(11) **EP 0 897 935 A2**
(43) Veröffentlichungstag der Anmeldung: **24.02.1999**
(21) Anmeldenummer: 98115042.8
(22) Anmeldetag: 11.08.1998
(51) Int. Cl.: C08F 20/36, C07D 519/00, C08L 33/14

(54) **Neue Polyacrylate mit sterisch gehinderten Piperidingruppen als Lichtschutzmittel für polymeres Material**

(30) Priorität: 18.08.1997 DE 19735764
(71) Anmelder: Clariant GmbH, 65929 Frankfurt am Main (DE)
(72) Erfinder: Mehrer, Mathias, Dr., 86456 Gablingen (DE)

(57) **Zusammenfassung**

Die vorliegende Erfindung bezieht sich auf neue Polyacrylate der allgemeinen Formel (II) 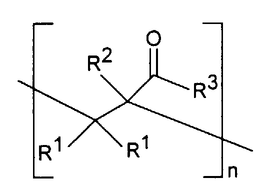 worin die Substituenten die in der Beschreibung definierte Bedeutung besitzen, oder auf Copolymere, die dieses Strukturelement enthalten, sowie auf ein Verfahren zu ihrer Herstellung.

Die neuen Polyacrylate sind hervorragende Stabilisatoren für organisches Material, insbesondere Lichtschutzmittel für Polymere.

## Beschreibung

Die Stabilisierung von polymeren Material gegen den zerstörerischen Einfluß energiereicher Strahlung ist Gegenstand intensiver Forschungen. In den letzten Jahren wurde eine Vielzahl von Lichtschutzmitteln auf Basis sterisch gehinderter Amine entwickelt, die die Stabilisierung von polymeren Material in ausgezeichneter Art und Weise gewährleisten. Dennoch leiden gerade niedermolekulare Stabilisatoren dieser Substanzklasse unter dem Nachteil der leichten Extrahierbarkeit sowie hoher Flüchtigkeit aus dem zu stabilisierenden polymeren Material, so daß nach einiger Anwendungszeit unerwünschte Zersetzung des Materials eintritt. Aus diesem Grund wurde eine beträchtliche Anzahl polymerer Stabilisatoren auf Basis sterisch gehinderter Amine hergestellt, die die dargestellten Nachteile nicht oder nur in geringerem Maße besitzen. Als exemplarische Beispiele seien Stabilisatoren erwähnt, wie sie z. B. in EP-A-93 693, DE-A-2 719 131, EP-A-343 717 und EP-A-402 889 beschrieben sind.

Die Aktivierung von Lichtstabilisatoren auf Basis sterisch gehinderter Amine (engl: hindered amine light stabilizers oder HALS) mittels einer reaktiven Epoxidfunktion wird z. B. von Luston und Vass, Makromol. Chem., Macromol. Symp. **27**, 231 (1989) oder in EP-A-402 889 beschrieben. EP-A-526 399 beschreibt die Bindung von solchen 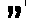 aktivierten" HALS-Molekülen an Fluorpolymere, welche Carboxylgruppen enthalten, während EP-A-001 835 die Umsetzung von mit Epoxidgruppen modifizierten sterisch gehinderten Piperidinen mit Dicarbonsäureanhydriden zu Polyestern lehrt. EP-A-638 597 beschreibt die Umsetzung von HALS mit Epichlorhydrin sowie die Addition von Acrylsäure oder deren Derivaten an die so entstehenden Zwischenstufen. Radikalische Polymerisation dieser Zwischenstufen führt dann zu Polyacrylaten, deren Molgewicht durch Variation der Polymerisationsbedingungen gesteuert werden kann.

Es besteht jedoch weiterhin ein steter Bedarf an neuen leistungsfähigeren Stabilisatoren, welche über verbesserte Lichtschutz- oder zusätzliche bessere Gebrauchseigenschaften verfügen.

Aufgabe der vorliegenden Anmeldung war es deshalb, neue Stabilisatoren bereitzustellen, die sich durch geringe Extrahierbarkeit bei hoher Lichtschutzwirkung auszeichnen.

Überraschenderweise wurde gefunden, daß man durch Umsetzung von Acrylsäurederivaten mit Verbindungen der Formel (I) 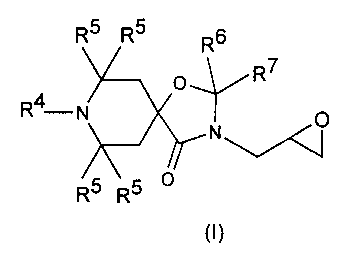 zu Stabilisatoren für Polymere mit hervorragender Lichtschutzwirkung und geringer Flüchtigkeit gelangt.

Gegenstand der Erfindung sind neue Polyacrylate der Formel (II), oder Copolymere, 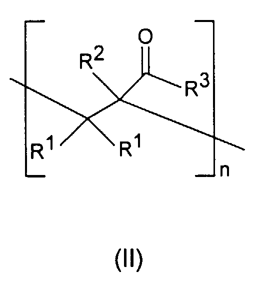 welche strukturelle Einheiten der Formel (II) auf der Polymerkette enthalten, welche über die Säurefunktion mit Molekülen der allgemeinen Formel (III), d. h. R³ = 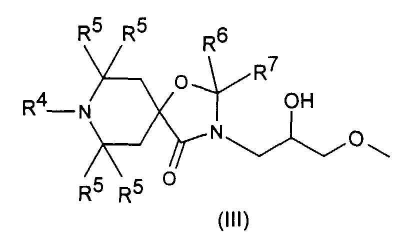 verknüpft sind.

In den Formeln bedeutet:
- n: 2 bis 1000, vorzugsweise 3 bis 100, insbesonders 5 bis 20,
- R¹ und R²: unabhängig voneinander insbesondere Wasserstoff, eine C₁-C₁₈-, vorzugsweise eine C₁-C₄-Alkylgruppe, insbesondere eine Methylgruppe oder Chlor, wobei nur ein Chloratom in der Struktureinheit vorkommt,
- R³: einen Rest der Formel (III), wobei
- R⁴: Wasserstoff, eine C₁-C₂₀-, vorzugsweise eine C₁-C₅-Alkylgruppe, insbesondere eine Methylgruppe, ein Sauerstoffradikal O*, -OH, -NO, -CH₂CN, Benzyl, Allyl, eine C₁-C₃₀-, vorzugsweise eine C₁-C₁₀-Alkyloxygruppe, eine C₅-C₁₂-, vorzugsweise eine C₅-C₆-Cycloalkyloxygruppe, eine C₃-C₁₀-, vorzugsweise eine C₃-C₆-Alkenylgruppe, eine C₃-C₆-Alkinylgruppe, eine C₁-C₁₀-, vorzugsweise eine C₁-C₅-Acylgruppe, Halogen, einen unsubstituierten oder durch C₁-C₄-, vorzugsweise durch C₁-C₂-Alkyl substituierten Phenylrest,
- R⁵: Wasserstoff oder eine C₁-C₁₂-Alkylgruppe, vorzugsweise eine C₁-C₄-Alkylgruppe, insbesondere eine Methylgruppe,
- R⁶ und R⁷: unabhängig voneinander ein Wasserstoffatom, eine C₁-C₁₈-, vorzugsweise eine C₁-C₈-, insbesondere eine C₁-C₄-Alkylgruppe oder zusammen mit dem sie verbindenden C-Atom einen Ring der Ringgröße 5 bis 13, vorzugsweise mit 6 bis 12, insbesondere mit 12 Ringgliedern oder zusammen mit dem sie bindenden C-Atom eine Gruppe der Formel (IV) 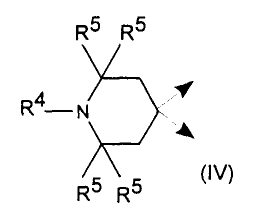 bedeutet, in denen die Reste R⁴ und R⁵ die obige Bedeutung haben.

Das zweimalige Vorkommen des Restes R¹ in Formel (II) heißt nicht, daß hier eine symmetrische Substitution vorliegt, vielmehr kann jeder der Reste R¹ unabhängig vom anderen Rest R¹ die oben genannte Bedeutung aufweisen.

Das mittlere Molekulargewicht Mₙ der auf diese Weise zugänglichen Polymere beträgt zwischen 1000 und 500000 g/mol.

Die Erfindung bezieht sich auch auf ein allgemeines Herstellverfahren von Verbindungen der Formel (II), sowie deren Verwendung zur Stabilisierung von polymeren organischen Material, insbesondere von Kunststoffen, Anstrichmitteln, Lacken und Ölen.

Die Herstellung der Verbindungen der Formel (II) basiert auf der Umsetzung von Verbindungen der allgemeinen Formel (I), beschrieben z.B. in EP-A-402 889, mit Acrylsäurederivaten der allgemeinen Formel (V) 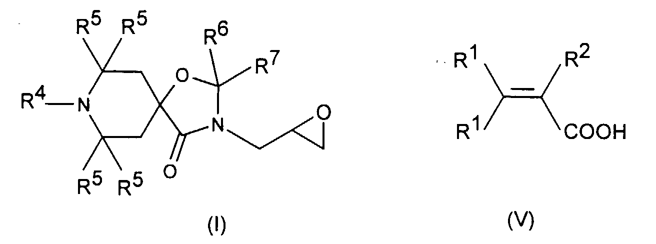 in der die Reste R¹, R², R⁴, R⁵, R⁶ und R⁷ die obengenannte Bedeutung aufweisen, zu Verbindungen der allgemeinen Formel (VI).

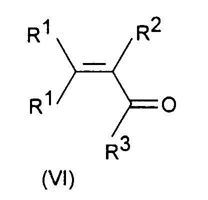

Diese acrylisch ungesättigten Verbindungen können dann auf an sich bekannte Weise zu den erfindungsgemäßen Verbindungen der Formel (II) polymerisiert werden.

Als Ausgangsmaterial gemäß der Formel (V) eignen sich z.B. Acrylsäure, 3-Alkyl- oder 3,3-Dialkylacrylsäure, Methacrylsäure, oder auch Itaconsäure, Glutaconsäure, Mesaconsäure und Crotonsäure.

Die Umsetzung von Verbindungen der Formeln (I) und (V) kann entweder mit oder ohne Lösemittel durchgeführt werden, wobei polare oder unpolare, inerte, besonders aber hochsiedende organische Lösemittel, z. B. aromatische oder aliphatische Kohlenwasserstoffe wie Ligroin, hochsiedender Petrolether, Toluol, Xylol, Mesitylen oder Decalin geeignet sind. Die Reaktionstemperatur kann zwischen Raumtemperatur und Rückflußtemperatur des entsprechenden Lösemittels gewählt werden; besonders bevorzugt ist hierbei Rückflußtemperatur. Die Reaktionsdauer beträgt zwischen 12 und 72 h. Die Reaktion wird bevorzugt unter einer Inertgasatmosphäre (Stickstoff oder Argon) durchgeführt, wobei Epoxide der Formel (I) und Säuren der Formel (V) im Molverhältnis von 10:1 bis 1:10, bevorzugt von 5:1 bis 1:5, insbesondere von 1:1 bis 1:3 eingesetzt werden. Die Reaktion wird vorzugsweise in Anwesenheit eines Katalysators, z.B. Tetraalkylammoniumhalogenid, bevorzugt Tetrabutyl-oder Tetraethylammoniumhalogenid oder quartären Phosphoniumsalzen, z. B. Ethyltriphenylphosphoniumbromid, durchgeführt. Der Katalysator wird zweckmäßig in einer Menge von 1 bis 5 Mol-% bezogen auf Verbindungen der Formel (I) eingesetzt. Die Aufarbeitung der entstandenen Verbindungen der allgemeinen Formel (VI) erfolgt nach gängigen Methoden wie z.B. Kristallisation oder Chromatographie. In einer bevorzugten Ausführungsvariante werden Verbindungen der Formel (VI) nach Entfernen des Lösemittels direkt der Polymerisation zugeführt. Die Herstellung der beanspruchten Verbindungen (Homopolymere der Formel (II)) erfolgt durch radikalische Polymerisation von Verbindungen der Formel (VI). Gegebenenfalls sind auch Copolymere durch gleichzeitige Polymerisation von Verbindungen der Formel (VI) und Acrylsäure- oder Methacrylsäurederivaten zugänglich. Solche hergestellten, beanspruchten Copolymere enthalten dann Einheiten der Formel (II) auf der Polymerkette. Die Polymerisation wird durch konventionelle Initiatoren, z.B. Azoverbindungen, insbesondere 2,2'-Azoisobuttersäurenitril (AIBN) oder Peroxiden, z.B. Benzoylperoxid oder Dibenzoylperoxid, eingeleitet. Der Initiator wird in einem Verhältnis von 0,1 bis 5 Mol-%, bevorzugt von 0,5 bis 3 Mol-%, bezogen auf die eingesetzten Verbindungen der Formel (VI), eingesetzt. Für die Kontrolle des Molgewichts können, falls erforderlich, noch Kettenübertragungsreagenzien wie z. B. Disulfide, Mercaptane oder Halogenide zugesetzt werden. Die Polymerisation wird in einer bevorzugten Herstellungsvariante in Lösung durchgeführt. Als Lösemittel eignen sich bevorzugt inerte, aromatische Kohlenwasserstoffe, insbesondere Xylol, Toluol oder Mesitylen; die Reaktionstemperatur bewegt sich zwischen 10°C und Rückflußtemperatur des entsprechenden Lösemittels, bevorzugt wird eine Temperatur von 50 bis 100 °C. Der vollständige Ausschluß von Luftsauerstoff ist zwingend notwendig und wird durch eine Inertgasatmosphäre gewährleistet.

Die erfindungsgemäßen Verbindungen eignen sich in hervorragender Weise zum Stabilisieren von organischen Material gegen die Einwirkung von Licht, Sauerstoff und Wärme. Sie werden dem zu stabilisierenden Material in einer Konzentration von 0,001 bis 5 Gew.-%, vorzugsweise von 0,02 bis 1 Gew.-%, bezogen auf das organische Material, vor, während oder nach seiner Herstellung, entweder alleine oder in Kombination mit weiteren Additiven zugesetzt. Unter organischem Material sind beispielsweise Vorprodukte für Kunststoffe, Lacke und Öle, insbesondere jedoch Kunststoffe, Anstrichmittel, Lacke und Öle selbst, zu verstehen.

Gegenstand der vorliegenden Erfindung ist außerdem ein gegen die Einwirkung von Licht, Sauerstoff und Wärme stabilisiertes organisches Material, insbesondere Kunststoffe, Anstrichmittel, Lacke und Öle, welches Verbindungen der Formel (II) in den oben angegebenen Konzentrationen enthält. Zu diesen Materialien gehören beispielsweise Stoffe wie sie in der deutschen Patentanmeldung Nr. 19 719 944.5 auf den Seiten 44 bis 50 beschrieben sind, worauf hier ausdrücklich Bezug genommen wird.

Das durch die erfindungsgemäßen Verbindungen der Formel (II) stabilisierte organische Material kann gegebenenfalls noch weitere Additive enthalten, beispielsweise Antioxidantien, Lichtstabilisierungsmittel, Metalldesaktivatoren, antistatische Mittel, flammhemmende Mittel, Pigmente und Füllstoffe. Antioxidantien und Lichtstabilisatoren, die neben den erfindungsgemäßen Verbindungen zugesetzt werden, sind beispielsweise Verbindungen auf der Basis sterisch gehinderter Amine oder sterisch gehinderter Phenole oder Schwefel oder Phosphor enthaltende Costabilisatoren. Als geignete zusätzliche in Kombination einsetzbare Additive sind beispielsweise Verbindungen, wie sie in der deutschen Patentanmeldung Nr. 19 719 944.5 auf den Seiten 51 bis 65 beschrieben sind, worauf hier ausdrücklich Bezug genommen wird.

Die Additive der allgemeinen Formel (II) werden nach den allgemein üblichen Methoden in die organischen Polymere eingearbeitet. Die Einarbeitung kann beispielsweise durch Einmischen oder Aufbringen der Verbindungen und gegebenenfalls weiterer Additive in oder auf das Polymere unmittelbar nach der Polymerisation oder in die Schmelze vor oder während der Formgebung erfolgen. Auch durch Aufbringen der gelösten oder dispergierten Verbindungen auf das Polymere direkt oder durch Einmischen in eine Lösung, Suspension oder Emulsion des Polymeren, gegebenenfalls unter nachträglicher Verdunstung des Lösemittels, kann die Einarbeitung erfolgen. Die Verbindungen sind auch wirksam, wenn sie in ein bereits granuliertes Polymer nachträglich in einem gesonderten Verarbeitungsschritt eingebracht werden. Die erfindungsgemäßen Verbindungen der Formel (II) können auch in Form eines Masterbatches, der diese Verbindungen beispielsweise in einer Konzentration von 1 bis 75, vorzugsweise 2,5 bis 30 Gew.-% enthält, den zu stabilisierenden Polymeren zugesetzt werden.

### Beispiele:

Die nachfolgenden Beispiele sollen die Erfindung näher erläutern, ohne diese in irgendeiner Weise näher einzuschränken. Alle hergestellten Edukte der Formel (VII) konnten durch ihre ¹H bzw ¹³C NMR-Spektren eindeutig charakterisiert werden. Einige charakteristische ¹³C-Signale dieser Verbindungen wurden tabellarisch (vgl. Tab. 1) zusammengefäßt. Die erfindungsgemäßen Polymere der Formel (II) wurden anhand ihrer C,H,N,O -Elementaranalysen charakterisiert; zur Bestimmung ihrer Eigenschaften wurden ihre Molmassenverteilungen, ermittelt durch das Verfahren der Gelpermeationschromatographie (GPC) sowie ihre Flüchtigkeiten, ermittelt durch die Methode der Thermogravimetrie (TGA) (gemessen in % bei einer gegebenen Temperatur), herangezogen.

### Verbindungen der Formel (VII):

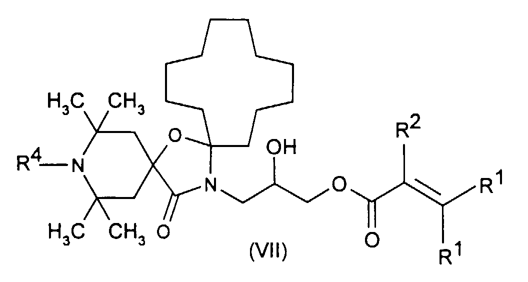

### Beispiel 1 (R¹ = H, R² = CH₃, R⁴ = H):

In einem Vierhalskolben mit Tropftrichter, Rückflußkühler, Innenthermometer und Stickstoffanschluß werden 42,0 g (0,1 mol) 2,2,4,4-Tetramethyl-20-(oxiranylmethyl)-7-oxa-3,20-di-azadispiro[5.1.11.2]heneicosan-21-on und 1,0 g (3,6 mmol) Tetrabutylammoniumchlorid (TEBA-chlorid) in 200 ml wasserfreiem Xylol vorgelegt und bei Raumtemperatur 20,6 g (0,24 mol) Methacrylsäure langsam zugetropft. Die weiße Suspension wird danach 12 h unter Rückfluß zum Sieden erhitzt, die Lösung abgekühlt und der Ansatz mit 100 ml 2n Natronlauge versetzt. Die Phasen werden getrennt und die organische Phase wird noch zweimal mit je 50 ml Wasser extrahiert, über Natriumsulfat getrocknet, vom Trockenmittel filtriert und das Lösemittel Xylol destillativ entfernt. Es verbleiben 49,9 g (98,6%) eines zähen, hellgelben Harzes, welches direkt ohne weitere Reinigung der Polymerisation zugeführt werden kann (charakt. spektroskop. Daten vgl. Tab. 1).

### Beispiel 2 (R¹ = H, R² = H, R⁴ = H):

Analog Beispiel 1 aus 42.0 g (0.10 mol) 2,2,4,4-Tetramethyl-20-(oxiranylmethyl)-7-oxa-3,20-diazadispiro[5.1.11.2]heneicosan-21-on, 1.0 g (3.6 mmol) TEBA-chlorid und 17.3 g (0.24 mol) Acrylsäure in 200 ml wasserfreiem Xylol. Nach Aufarbeitung verbleiben 48.4g (98.4%) eines leicht gelben, zähen Harzes, welches direkt weiter eingesetzt wird (charakt. spektroskop. Daten vgl. Tab. 1).

### Beispiel 3 (R¹ = H, R² = H, R⁴ = CH₃):

Analog Beispiel 1 aus 43,4 g (0,10 mol) 2,2,3,4,4-Pentamethyl-20-(oxiranylmethyl)-7-oxa-3,20-diazadispiro[5.1.11.2]heneicosan-21-on, 1,0 g (3,6 mmol) TEBA-chlorid und 17,3 g (0,24 mol) Acrylsäure in 200 ml wasserfreiem Xylol.Es verbleiben 50,1 g (99,0%) eines leicht gelben, zähen Harzes zurück, welches direkt weiter umgesetzt werden kann (charakt. spektroskop. Daten vgl. Tab. 1).

### Beispiel 4 (R¹ = H, R² = H, R⁴ = COCH₃):

Analog Beispiel 1 aus 46,2 g (0,10 mol) 3-Acetyl-2,2,4,4-tetramethyl-20-(oxiranylmethyl)-7-oxa-3,20-diazadispiro[5.1.11.2[heneicosan-21-on, 1,0 g (3.6 mmol) TEBA-chlorid und 17,3 g (0,24 mol) Acrylsäure in 200 ml wasserfreiem Xylol. Es verbleiben 53,6 g (100%) eines gelben klebrigen Hartes, welches direkt weiter eingesetzt wird (charakt. spektroskop. Daten vgl. Tab. 1).

### Beispiel 5 (R¹ = H, R² = CH₃, R⁴ = CH₃):

Analog Beispiel 1 aus 43,4 g (0,10 mol) 2,2,3,4,4-Pentamethyl-20-(oxiranylmethyl)-7-oxa-3,20-diazadispiro[5.1.11.2]heneicosan-21-on, 1,0 g (3,6 mmol) TEBA-chlorid und 20,6 g (0,24 mol) Methacrylsäure in 200 ml wasserfreiem Xylol. Nach der Reaktion und Aufarbeitung wird das gelbe, zähe Harz aus 200 ml n-Hexan umkristallisiert. Der Schmelzpunkt des isolierten Produkts (33,0 g; 63,5%) beträgt 114-116°C (charakt. spektroskop. Daten vgl. Tab. 1).

### Beispiel 6 (R¹ = H, R² = CH₃, R⁴ = COCH₃):

Analog Beispiel 1 aus 46,2 g (0,10 mol) 3-Acetyl-2,2,4,4-tetramethyl-20-(oxiranylmethyl)-7-oxa-3,20-diazadispiro[5.1.11.2]heneicosan-21-on, 1,0 g (3,6 mmol) TEBA-chlorid und 20,6 g (0,24 mol) Methacrylsäure in 200 ml wasserfreiem Xylol. Es verbleiben 54,0 g (98,5%) eines gelben klebrigen Harzes, welches direkt weiter eingesetzt wird (charakt. spektroskop. Daten vgl. Tab. 1).

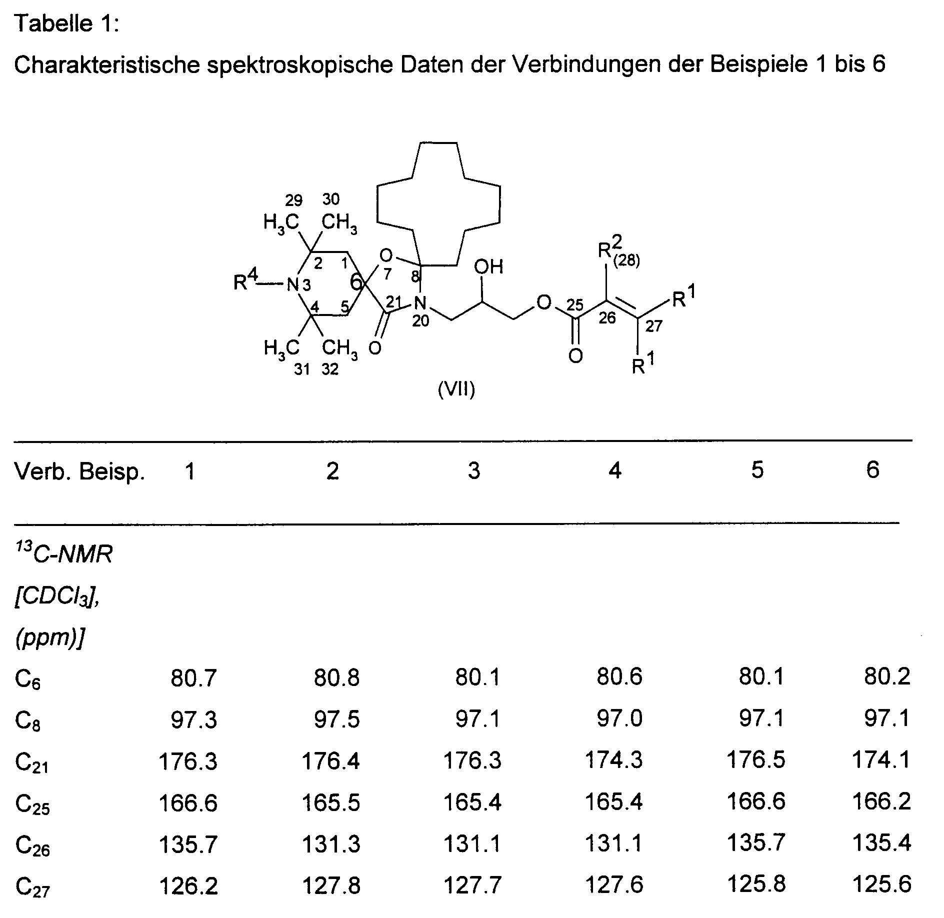

### Polymere Verbindungen der Formel (II):

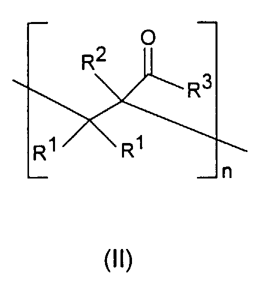

mit R³ ein Rest der Formel (VIII)

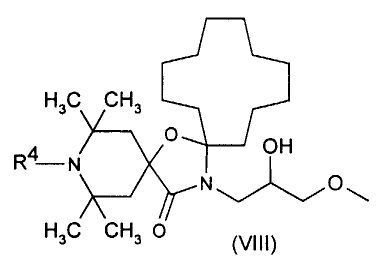

### Beispiel 7 (R¹ = H, R² = CH₃, R⁴ = H):

In einem Vierhalskolben mit Tropftrichter, Rückflußkühler, Innenthermometer und Stickstoffanschluß werden 49,1 g (0,097 mol) der Verbindung aus Beispiel 1 in 350 ml wasserfreiem Toluol vorgelegt und bei Raumtemperatur 350 mg (2,13 mmol) 2,2'-Azoisobuttersäurenitril (AIBN) zugesetzt. Das Reaktionsgemisch wird 32 h bei 70°C gehalten, danach 1,0 g Aktivkohle hinzugefügt, die Lösung vom Feststoff filtriert und das entstandene Polymer mit n-Hexan gefällt. Das Material wird durch Filtration isoliert; zur Reinigung erneut in Toluol gelöst und nochmals mit n-Hexan gefällt. Das Polymer besitzt einen Schmelzbereich von 169-188°C.

| Elementaranalyse: | | | | | |
|---|---|---|---|---|---|
| (C₂₉H₅₀N₂O₅)n | Berechnet | %C 68.77 | %H 9.88 | %N 5.53 | %O 15.81 |
| (506.0)ₙ | Gefunden | %C 67.88 | %H 10.10 | %N 5.52 | %O 16.25 |

- TGA (N₂, 2°C/min): 8.2 % Gewichtsverlust bei 300°C
- GPC:: Mₙ = 2200
M_{w}= 11800

### Beispiel 8 (R¹ = H, R² = H, R⁴ = H):

Analog Beispiel 7 aus 47,7 g (0,097 mol) der Verbindung aus Beispiel 2 und 350 mg (2,13 mmol) AIBN in 350 ml wasserfreiem Toluol. Reinigung des Produkts durch Lösen in Toluol und Fällen des Polymers mit n-Hexan. Man erhält 28,6 g (60%) eines hellbeigen Pulvers vom Schmelzpunkt 157°C.

| Elementaranalyse: | | | | | |
|---|---|---|---|---|---|
| (C₂₈H₄₈N₂O₅)ₙ | Ber. | %C 68.29 | %H 9.76 | %N 5.69 | %O16.26 |
| (492.0)ₙ | Gef. | %C 68.05 | %H 10.03 | %N 5.50 | %O16.35 |

- TGA (N₂, 2°C/min): 13.4 % Gewichtsverlust bei 300°C
- GPC:: Mₙ = 5500
M_{w} = 11990

### Beispiel 9 (R¹ = H, R² = H, R⁴ = CH₃):

Analog Beispiel 7 aus 50,1 g (0,1 mol) der Verbindung aus Beispiel 3 und 330 mg (2,01 mmol) AIBN in 330 ml wasserfreiem Toluol. Das Polymer wird nach der Reaktion mit Acetonitril gefällt, zur Reinigung erneut in Toluol gelöst und nochmals mit Acetonitril gefällt. Nach Trocknen des Produkts im Vakuum erhält man 23,8 g (47,5%) eines hellen Pulvers vom Schmelzbereich 153 bis 180°C.

| Elementaranalyse: | | | | | |
|---|---|---|---|---|---|
| (C₂₉H₅₀N₂O₅)ₙ | Ber. | %C 68.77 | %H 9.88 | %N 5.53 | %O 15.81 |
| (506.0)ₙ | Gef. | %C 67.81 | %H 10.06 | %N 5.49 | %O 16.44 |

- TGA (N₂, 2°C/min): 12.5 % Gewichtsverlust bei 300°C
- GPC:: Mₙ = 2800
M_{w} = 6100

### Beispiel 10 (R¹ = H, R² = H, R⁴ = COCH₃):

Analog Beispiel 7 aus 53,4 g (0,1 mol) der Verbindung aus Beispiel 4 und 330 mg (2,01 mmol) AIBN in 350 ml wasserfreiem Toluol. Nach Isolierung und Reinigung des Polymers durch Lösen in Toluol und Fällen mit Acetonitril erhält man 28,3 g (53%) eines hellen Pulvers vom Schmelzbereich 190 bis 250°C.

| Elementaranalyse: | | | | | |
|---|---|---|---|---|---|
| (C₃₀H₅₀N₂O₆)ₙ | Ber. | %C 67.42 | %H 9.36 | %N 5.24 | %O 17.98 |
| (534.0)ₙ | Gef. | %C 66.32 | %H 9.40 | %N 5.05 | %O 19.34 |

- TGA (N₂, 2°C/min): 7.2 % Gewichtsverlust bei 300°C
- GPC:: Mₙ = 8300
M_{w} = 70000

### Beispiel 11 (R¹ = H, R² = CH₃, R⁴ = CH₃):

Analog Beispiel 7 aus 50,0 g (0,096 mol) der Verbindung aus Beispiel 5 und 330 mg (2,01 mmol) AIBN in 350 ml wasserfreiem Toluol. Nach Isolierung und Reinigung des Polymers durch Lösen in Toluol und Fällen mit Acetonitril erhält man 19,0 g (38 %) eines hellen Pulvers vom Schmelzbereich 185 bis 196°C.

| Elementaranalyse: | | | | | |
|---|---|---|---|---|---|
| (C₃₀H₅₂N₂O₅)ₙ | Ber. | %C 69.23 | %H 10.00 | %N 5.38 | %O 15.38 |
| (520.0)ₙ | Gef. | %C 68.09 | %H 10.20 | %N 5.52 | %O 16.26 |

- TGA (N₂, 2°C/min): 9.2 % Gewichtsverlust bei 300°C
- GPC:: Mₙ = 4000
M_{w} = 11200

### Beispiel 12 (R¹ = H, R² = CH₃, R⁴ = COCH₃):

Analog Beispiel 7 aus 54,0 g (0,098 mol) der Verbindung aus Beispiel 6 und 330 mg (2,01 mmol) AIBN in 350 ml wasserfreiem Toluol. Nach Isolierung und Reinigung des Polymers durch Lösen in Toluol und Fällen mit n-Hexan erhält man 21,1 g (39 %) eines hellen Pulvers vom Schmelzpunkt 120°C.

| Elementaranalyse: | | | | | |
|---|---|---|---|---|---|
| (C₃₁H₅₂N₂O₆)ₙ | Ber. | %C 67.88 | %H 9.49 | %N 5.11 | %O 17.52 |
| (548.0)ₙ | Gef. | %C 67.94 | %H 9.97 | %N 5.45 | %O 16.47 |

- TGA (N₂, 2°C/min): 9.0 % Gewichtsverlust bei 300°C
- GPC:: Mₙ = 1600
M_{w} = 4800

### Beispiel 13: Lichtstabilisierende Wirkung in Polypropylenfolien:

100 Gewichtsteile unstabilisiertes Polypropylen ®Hostalen PPK werden zusammen mit 0,2 Gewichtsteilen Calciumstearat, 0,1 Gewichtsteilen Tris-(2,4-di-tert-butylphenyl)-phosphit (®Hostanox PAR 24) und 0,2 Gewichtsteilen des zu prüfenden Stabilisators in einem Brabender-Mischer 10 min bei 200°C und 20 U/min geknetet. Aus dieser Mischung wurde bei 190°C eine 100 µm starke Preßfolie hergestellt und die auf diese Weise erhaltenen Prüfkörper in einem Bewitterungsgerät (®Xenotest 1200) belichtet. Als getestetes Kriterium der Stabilität der Folie wurde die Zeit bis zur Zunahme des Carbonylindexes um 1,0 Einheiten herangezogen. Die Zunahme des Carbonylindexes mit der Zeit ist hierbei ein Maß für die Abnahme der mechanischen Eigenschaften der betreffenden Folie. Der Carbonylindex CO wurde gemäß der Formel CO = E₁₇₂₀/E₂₀₂₀ bestimmt. Zu Vergleichszwecken wurde eine Folie unter den gleichen Bedingungen, jedoch ohne den Zusatz der erfindungsgemäßen Stabilisatoren getestet. Die Versuchsergebnisse sind in Tab. 1 zusammengestellt:

**Tabelle 1**

| Belichtungsdauer bis zur Zunahme des Carbonylindex um 1,0 Einheiten: | |
|---|---|
| Stabilisator | Belichtungsdauer |
| keiner | 191 h |
| aus Beispiel 7 | < 1320 h |
| aus Beispiel 8 | > 1227 h < 1320 h |
| aus Beispiel 9 | > 1227 h < 1320 h |
| aus Beispiel 11 | > 898 h < 1133 h |

Die Ergebnisse der künstlichen Bewitterung unterstreichen die sehr gute Lichtschutzwirkung der erfindungsgemäßen Stabilisatoren.

## Patentansprüche

1. Neue Polyacrylate der allgemeinen Formel (II) 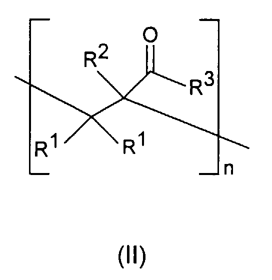 oder Copolymere, welche strukturelle Einheiten der Formel (II) auf der Polymerkette enthalten, dadurch gekennzeichnet, daß
n 2 bis 1000,
R¹ und R² unabhängig voneinander Wasserstoff, eine C₁-C₁₈-Alkylgruppe oder Chlor bedeutet, wobei nur ein Chloratom in der Struktureinheit vorkommt,
R³ ein Rest der Formel (III) 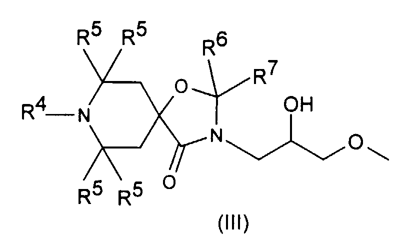 ist, wobei
R⁴ Wasserstoff, eine C₁-C₂₀-Alkylgruppe, ein Sauerstoffradikal O*, -OH, -NO, -CH₂CN, Benzyl, Allyl, C₁-C₃₀-Alkyloxy, C₅-C₁₂-Cycloalkyloxy, C₃-C₁₀-Alkenyl, C₃-C₆-Alkinyl, C₁-C₁₀-Acyl, Halogen, einen unsubstituierten oder durch C₁-C₄-Alkyl substituierten Phenylrest bedeutet,
R⁵ Wasserstoff oder eine C₁-C₁₂-Alkylgruppe ist,
R⁶ und R⁷ unabhängig voneinander ein Wasserstoffatom, eine C₁-C₁₈-Alkylgruppe oder zusammen mit dem sie verbindenden C-Atom einen Ring der Ringgröße 5 bis 13, oder zusammen mit dem sie bindenden C-Atom eine Gruppe der Formel (IV) 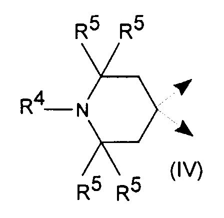 in denen die Reste R⁴ und R⁵ die obige Bedeutung haben, bedeutet.

2. Polyacrylate gemäß Anspruch 1, dadurch gekennzeichnet, daß
n 3 bis 100,
R¹ und R² unabhängig voneinander Wasserstoff oder C₁-C₄-Alkyl,
R³ ein Rest der Formel (III) ist, wobei
R⁴ Wasserstoff, C₁-C₅-Alkyl, ein Sauerstoffradikal O*, -OH, -NO, -CH₂CN, Benzyl, Alkyl, C₁-C₁₀-Alkyloxy, C₅-C₆-Cycloalkyloxy, C₃-C₆-Alkenyl, C₃-C₆-Alkinyl, C₁-C₅-Acyl, einen durch C₁-C₂-Alkyl substituierten Phenylrest bedeutet,
R⁵ Wasserstoff oder C₁-C₄-Alkyl,
R⁶ und R⁷ unabhängig voneinander ein Wasserstoffatom oder C₁-C₈-Alkyl oder zusammen mit dem sie verbindenden C-Atom einen Ring der Ringgröße 6 bis 12 oder zusammen mit dem sie bindenden C-Atom eine Gruppe der Formel (IV) 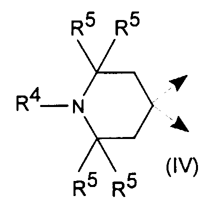 in denen die Reste R⁴ und R⁵ die obige Bedeutung haben, bedeutet.

3. Polyacrylate gemäß Anspruch 1 und 2, dadurch gekennzeichnet,
n 5 bis 20,
R¹ und R² unabhängig voneinander Wasserstoff oder Methyl bedeutet,
R³ ein Rest der Formel (III) ist, wobei
R⁴ Wasserstoff, C₁-C₅-Acyl, Methyl,
R⁵ Wasserstoff oder Methyl, und
R⁶ und R⁷ unabhängig voneinander Wasserstoff oder C₁-C₄-Alkyl oder zusammen mit dem sie verbindenden C-Atom einen Ring der Ringgröße 12 bedeuten.

4. Verfahren zur Herstellung der Verbindungen (II), dadurch gekennzeichnet, daß Verbindungen der Formel (I) mit Acrylsäureverbindungen der Formel (V) zu Verbindungen der Formel (VI) umgesetzt und dann polymerisiert 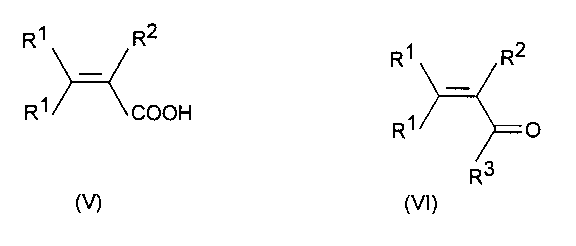 werden.

5. Verwendung der Verbindungen der Formel (II) zum Stabilisieren von organischen Material, insbesondere als Lichtschutzmittel für Kunststoffe Anstrichmittel, Lacke und Öle.

6. Verfahren zum Stabilisieren von polymeren Material gegen den zerstörerischen Abbau durch Licht und Wärme, dadurch gekennzeichnet, daß man dem zu stabilisierenden Material eine Verbindung der Formel (II) nach Anspruch 1 bis 3 in einer Konzentration von 0,001 bis 5, vorzugsweise von 0,02 bis 1 Gew.-%, bezogen auf das organische Material, vor, während oder nach seiner Herstellung zusetzt.

7. Stabilisiertes organisches Material, welches mindestens eine der Verbindungen der Formel (II) gemäß Anspruch 1 enthält.
